# FASCICULE DE BREVET EUROPEEN

(11) **EP 1 404 258 B1**
(45) Date de publication et mention de la délivrance du brevet: **25.06.2008**
(21) Numéro de dépôt: 02760382.8
(22) Date de dépôt: 08.07.2002
(51) Int. Cl.: A61F 2/44

(54) **IMPLANT INTERSOMATIQUE POUR VERTEBRES CERVICALES**
HALSWIRBELKÄFIGIMPLANTAT
INTERVERTEBRAL IMPLANT FOR CERVICAL VERTEBRAE

(30) Priorité: 10.07.2001 FR 0109428
(43) Date de publication de la demande: 07.04.2004
(73) Titulaire: Vitatech, F-74970 Marignier (FR)
(72) Inventeur: GRADEL, Thomas, F-74130 AYZE (FR)
(74) Mandataire: Poncet, Jean-François
(86) Numéro de dépôt international: PCT/FR2002/002377
(87) Numéro de publication internationale: WO 2003/007841

(56) Documents cités:
- WO-A-01/28463
- FR-A- 2 795 945
- FR-A- 2 808 673
- US-A- 5 888 227

## Description

### DOMAINE TECHNIQUE DE L'INVENTION

La présente invention concerne les implants intersomatiques creux pour stabilisation de la colonne vertébrale, en particulier pour la stabilisation de vertèbres cervicales, destinés à former une cale à insérer entre les plateaux en vis à vis de deux vertèbres cervicales voisines, de manière à maintenir un espace discal constant.

Le document EP 0 307 241 A décrit un exemple d'implant intersomatique ayant la forme d'une cage parallélépipédique. Une telle forme de cage n'est pas bien adaptée à une utilisation dans la colonne vertébrale, notamment par le fait que les arêtes longitudinales d'une telle cage risquent de léser la moelle épinière ou les racines lors de l'insertion de l'implant entre les vertèbres adjacentes. On constate également une relative inefficacité du maintien des vertèbres adjacentes, vraisemblablement à cause de la forme parallélépipédique de la cage.

On a également proposé un implant intersomatique de forme parallélépipédique dans lequel les grandes faces supérieure et inférieure sont planes et forment entre elles un angle ouvert vers l'avant, pour maintenir les vertèbres adjacentes selon un angle physiologique approprié de lordose. La forme de l'implant nécessite de réaliser des évidements conséquents dans les plateaux vertébraux, lors de la pose, ce qui crée un traumatisme inapproprié.

Le document US 5 888 227 A décrit un implant intersomatique à paroi périphérique entourant une grande cavité intérieure unique. Les faces supérieure et inférieure sont divergentes vers l'avant et présentent une double courbure antéro-postérieure et transversale. La structure présente une résistance mécanique insuffisante.

### EXPOSE DE L'INVENTION

Le problème proposé par la présente invention est de définir une nouvelle structure d'implant intersomatique qui permette de réduire très sensiblement les risques de lésions nerveuses lors de la pose, qui permette d'assurer un meilleur positionnement et un meilleur blocage des vertèbres l'une avec l'autre, qui minimise le traumatisme osseux lors de la pose, et qui assure une résistance mécanique améliorée et un bon accrochage osseux pour faciliter la fusion entre deux vertèbres adjacentes.

Pour atteindre ces objets ainsi que d'autres, l'implant intersomatique selon l'invention, permettant la stabilisation de vertèbres cervicales, comprend un corps en matériau solide biocompatible ayant une forme générale parallélépipédique limitée par deux grandes faces supérieure et inférieure convexes légèrement divergentes vers l'avant, par deux faces latérales opposées, et par deux faces antérieure et postérieure, avec au moins une cavité intérieure mettant en communication des orifices prévus sur les grandes faces supérieure et inférieure ; le corps est une structure creuse à plusieurs cavités internes délimitées par des parois, avec un trou vertical médian traversant entouré d'une paroi cylindrique intermédiaire elle-même reliée à une paroi périphérique tubulaire d'axe vertical par des croisillons radiaux définissant des cavités internes périphériques traversantes ouvertes sur les grandes faces supérieure et inférieure.

Une telle structure à faces convexes minimise le traumatisme des tissus environnants, tout en assurant une stabilisation de l'implant qui évite sa migration latérale et sa migration antéro-postérieure.

Et la structure alvéolée augmente sensiblement la résistance mécanique à l'écrasement, et permet de réaliser l'implant avec des parois relativement fines, ou en un matériau présentant une résistance mécanique intrinsèque réduite.

De préférence, les grandes faces supérieure et inférieure comportent des nervures et rainures transversales, de façon à réduire encore les risques de migration ultérieure de l'implant parallèlement au plateau des vertèbres.

Selon un mode de réalisation avantageux, les grandes faces supérieure et inférieure convexes présentent une première courbure convexe dans la direction antéro-postérieure et une seconde courbure convexe dans la direction transversale.

De préférence, la paroi périphérique tubulaire comporte des trous latéraux, permettant la fusion latérale des tissus osseux.

De même, le cylindre intermédiaire comporte de préférence des trous périphériques mettant en communication le trou vertical médian avec les cavités périphériques traversantes, permettant de solidariser entre elles les parties de greffon engagées dans les diverses cavités de l'implant.

On peut avantageusement prévoir, sur la face antérieure de paroi périphérique tubulaire, un trou fileté de préhension conformé pour recevoir un outil de pose manipulable par l'utilisateur.

On peut réaliser l'implant en un matériau biocompatible solide tel que le titane, ou avantageusement en un polymère de type PEEK distribué par la société VICTREX, qui présente l'avantage d'être radiotransparent.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles:
- la figure 1 est une vue en perspective d'un implant intersomatique selon un mode de réalisation de la présente invention ;
- la figure 2 est une vue de dessus de l'implant de la figure 1, pivoté de 180° autour de l'axe du trou vertical médian ;
- la figure 3 est une vue de face de l'implant de la figure 1, montrant la face antérieure ; et
- la figure 4 est une vue de côté de l'implant de la figure 1.

### DESCRIPTION DES MODES DE REALISATION PREFERES

Dans le mode de réalisation illustré sur les figures, l'implant intersomatique selon l'invention comprend un corps en matériau solide biocompatible ayant une forme générale parallélépipédique aplatie, limitée par deux grandes faces supérieure 1 et inférieure 2, par deux faces latérales opposées 3 et 4, par une face postérieure 5 et par une face antérieure 6. La hauteur de l'implant, ou distance séparant les grandes faces supérieure 1 et inférieure 2, est plus petite que la largeur séparant les faces latérales opposées 3 et 4, et est également plus petite que la profondeur séparant les faces postérieure 5 et antérieure 6.

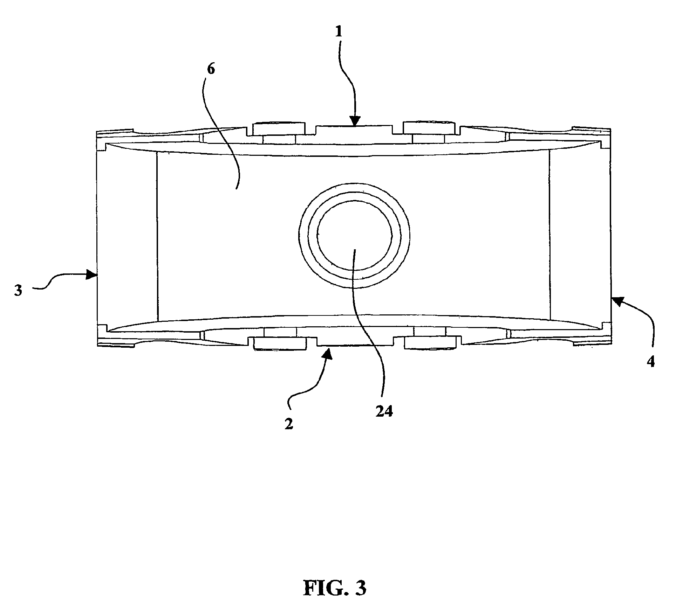

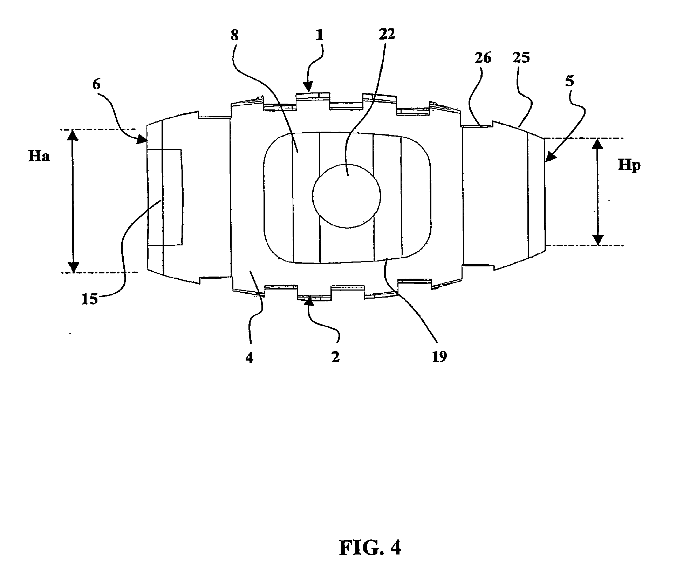

Les grandes faces supérieure 1 et inférieure 2 sont convexes, en présentant une première courbure convexe dans la direction antéro-postérieure, et une seconde courbure convexe dans la direction transversale. Ainsi, sur la figure 3, on distingue la seconde courbure convexe de direction transversale des deux grandes faces 1 et 2. De même, sur la figure 4, on distingue la première courbure convexe dans la direction antéro-postérieure.

En se référant à la figure 4 en vue de côté, on voit que les grandes faces supérieure 1 et inférieure 2 sont légèrement divergentes vers l'avant, c'est-à-dire en direction de la face antérieure : la hauteur Ha au voisinage de la face antérieure 6 est plus grande que la hauteur Hp au voisinage de la face postérieure 5.

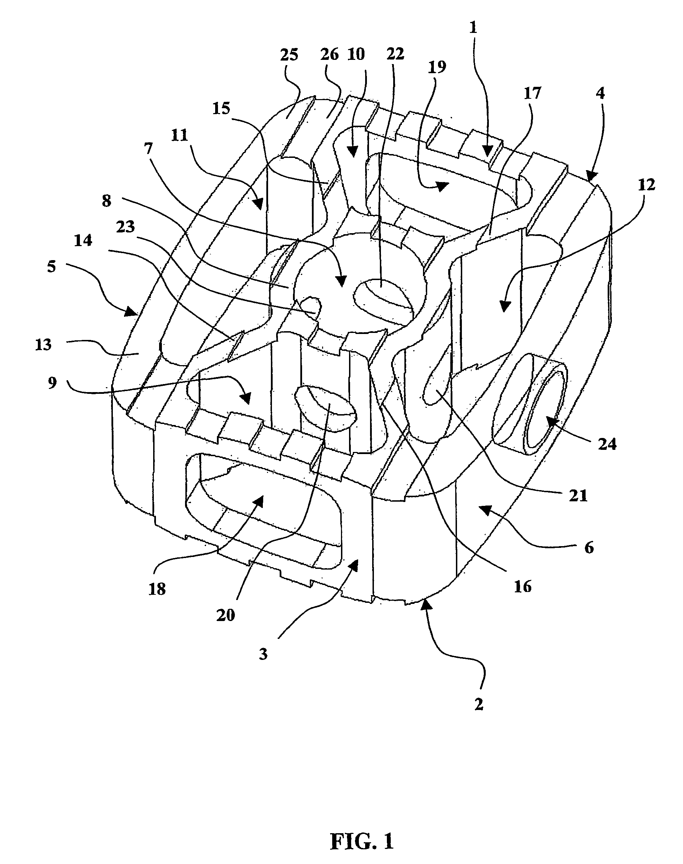

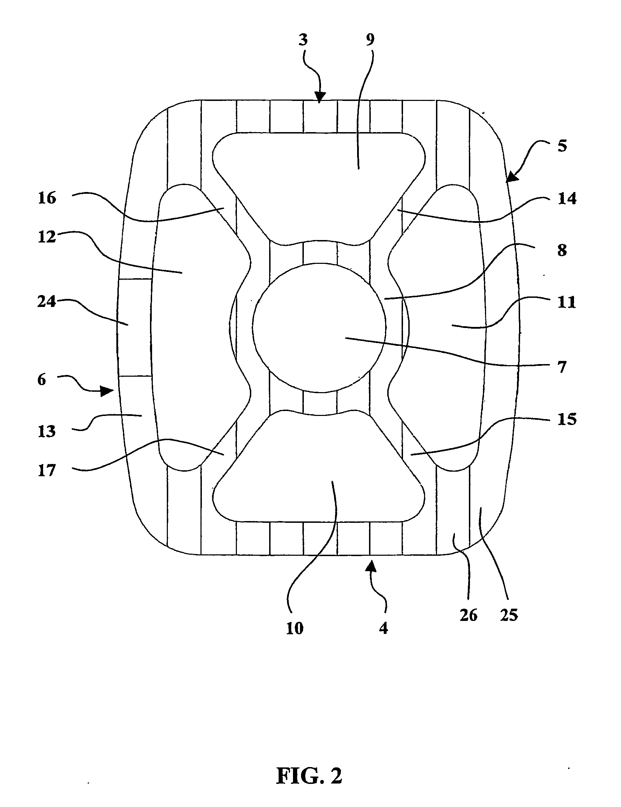

L'implant selon l'invention comprend des cavités intérieures mettant en communication des orifices prévus sur les grandes faces supérieure 1 et inférieure 2. Ainsi, sur les figures 1 et 2, on distingue une cavité interne centrale, formée d'un trou vertical médian 7 traversant, entourée d'une paroi cylindrique intermédiaire 8 elle-même entourée d'une succession de cavités périphériques traversantes 9, 10, 11 et 12 ouvertes sur les grandes faces supérieure 1 et inférieure 2. Les cavités périphériques traversantes 9-12 sont entourées d'une paroi périphérique tubulaire 13 d'axe vertical et formant les faces latérales opposées 3 et 4 et les faces postérieure 5 et antérieure 6.

La paroi cylindrique intermédiaire 8 est reliée à la paroi périphérique tubulaire 13 par des croisillons radiaux 14, 15, 16 et 17, définissant les cavités périphériques traversantes 9-12.

La paroi périphérique tubulaire 13 comporte des trous latéraux 18 et 19, occupant une grande partie des faces latérales opposées 3 et 4.

La paroi cylindrique intermédiaire 8 comporte des trous périphériques 20, 21, 22 et 23 mettant en communication le trou vertical médian 7 avec les cavités périphériques traversantes 9-12.

Dans la réalisation avantageuse illustrée sur les figures, les cavités périphériques traversantes 9-12 sont chacune au regard de l'une des faces latérales, antérieure ou postérieure. En d'autres termes, les croisillons radiaux 14-17 relient chacun la paroi cylindrique intermédiaire 8 avec l'un des coins de la paroi périphérique tubulaire 13.

La paroi périphérique tubulaire 13 comporte, sur sa face antérieure 6, un trou fileté de préhension 24, conformé pour coopérer avec un outil de préhension.

Egalement, les grandes faces supérieure 1 et inférieure 2 comportent des nervures et des rainures transversales. Par exemple, sur les figures 1 et 2, on a repéré la nervure transversale 25 voisine de l'extrémité postérieure de la grande face supérieure 1, et une rainure transversale 26 adjacente.

Le corps de l'implant peut être réalisé en titane, présentant ainsi une bonne résistance mécanique tout en étant biocompatible.

En alternative, le corps de l'implant peut avantageusement être en un polymère de type PEEK, présentant l'avantage de pouvoir être moulé, d'être radiotransparent, et de présenter de bonnes propriétés de biocompatibilité et de résistance mécanique dans la forme choisie.

Lors de l'utilisation, avant d'engager l'implant entre deux vertèbres adjacentes, on réalise dans les plateaux respectifs de chaque vertèbre un logement peu profond conformé pour recevoir les grandes faces supérieure 1 et inférieure 2 de l'implant. On introduit dans les cavités internes 7, 9-12 de l'implant des greffons d'os spongieux prélevés dans une autre partie du corps du patient. On écarte ensuite les deux vertèbres adjacentes, par des moyens appropriés, et l'on insère axialement l'implant entre les deux vertèbres en le maintenant avec un outil adapté pour le tenir par le trou fileté de préhension 24. On relâche les vertèbres. La forme biconvexe de l'implant donne aux vertèbres adjacentes une assise efficace, assurant une bonne stabilisation mécanique des vertèbres l'une par rapport à l'autre et avec l'implant, en reproduisant l'angle morphologique normal tel qu'une lordose anatomique. Les nervures et rainures transversales 25, 26 interdisent le déplacement axial de l'implant. Elles interdisent également la rotation de l'implant selon un axe vertical.

Après la pose de l'implant, les greffons d'os spongieux sont en contact avec l'os des vertèbres inférieure et supérieure, de façon à assurer progressivement la fusion des vertèbres l'une à l'autre. La taille importante des ouvertures supérieures et inférieures dans les grandes faces supérieure 1 et inférieure 2 de l'implant favorise cette fusion, en assurant un bon contact entre les greffons internes et l'os des deux vertèbres.

Toutefois, malgré la présence des larges ouvertures supérieure et inférieure de contact, l'implant présente de bonnes surfaces d'appuis supérieure et inférieure, réparties sur toute sa dimension, grâce notamment à la présence de la paroi cylindrique intermédiaire 8 et des croisillons radiaux 14-17, assurant un appui efficace et peu traumatisant sur les plateaux des vertèbres adjacentes.

On conçoit que la forme particulière de l'implant tel qu'illustré sur les figures assure une grande stabilité de la structure, et un maintien efficace des vertèbres l'une par rapport à l'autre.

La présente invention n'est pas limitée aux modes de réalisation qui ont été explicitement décrits, mais elle en inclut les diverses variantes et généralisations contenues dans le domaine des revendications ci-après.

## Revendications

1. - Implant intersomatique pour stabilisation de vertèbres cervicales, comprenant un corps en matériau solide biocompatible ayant une forme générale parallélépipédique limitée par deux grandes faces supérieure (1) et inférieure (2) convexes légèrement divergentes vers l'avant, par deux faces latérales opposées (3, 4), et par deux faces postérieure (5) et antérieure (6), avec au moins une cavité intérieure mettant en communication des orifices prévus sur les grandes faces supérieure (1) et inférieure (2), **caractérisé en ce que** le corps est une structure creuse à plusieurs cavités internes délimitées par des parois, avec un trou vertical médian (7) traversant entouré d'une paroi cylindrique intermédiaire (8) elle-même reliée à une paroi périphérique tubulaire (13) d'axe vertical par des croisillons radiaux (14-17) définissant des cavités internes périphériques traversantes (9-12) ouvertes sur les grandes faces supérieure (1) et inférieure (2).

2. - Implant intersomatique selon la revendication 1, **caractérisé en ce que** les grandes faces supérieure (1) et inférieure (2) comportent des nervures (25) et des rainures (26) transversales.

3. - Implant intersomatique selon l'une des revendications 1 ou 2, **caractérisé en ce que** les grandes faces supérieure (1) et inférieure (2) présentent une première courbure convexe dans la direction antéro-postérieure et une seconde courbure convexe dans la direction transversale.

4. - Implant intersomatique selon la revendication 3, **caractérisé en ce que** la paroi périphérique tubulaire (13) comporte des trous latéraux (18, 19).

5. - Implant intersomatique selon l'une des revendications 3 ou 4, **caractérisé en ce que** la paroi cylindrique intermédiaire (8) comporte des trous périphériques (20-23) mettant en communication le trou vertical médian (7) avec les cavités périphériques traversantes (9-12).

6. - Implant intersomatique selon l'une quelconque des revendications 3 à 5, **caractérisé en ce que** la paroi périphérique tubulaire (13) comporte, sur sa face antérieure (6), un trou fileté de préhension (24).

7. - Implant intersomatique selon l'une quelconque des revendications 1 à 6, **caractérisé en ce que** le matériau biocompatible solide est le titane.

8. - Implant intersomatique selon l'une quelconque des revendications 1 à 6, **caractérisé en ce que** le matériau biocompatible solide est un polymère de type PEEK.

## Claims

1. Intervertebral implant for stabilising cervical vertebrae, comprising a body made of a biocompatible solid material having a parallelepipedal overall shape bounded by two convex large faces, namely a top face (1) and a bottom face (2), which diverge slightly forwards, by two opposed lateral faces (3, 4) and by two faces, one posterior (5) and one anterior (6), with at least one internal cavity placing orifices made on the top (1) and bottom (2) large faces in communication with each other, **characterised in that** the body is a hollow structure with several internal cavities bounded by walls, with a central vertical through-hole (7) surrounded by an intermediate cylindrical wall (8) itself connected to a tubular peripheral wall (13) of vertical axis by radial crosspieces (14-17) defining peripheral internal through-cavities (9-12) that open onto the top (1) and bottom (2) large faces.

2. Intervertebral implant according to claim 1, **characterised in that** the top (1) and bottom (2) large faces have transverse ribs (25) and grooves (26).

3. Intervertebral implant according to one of claims 1 and 2, **characterised in that** the top (1) and bottom (2) large faces have a first curvature that is convex in the anterior-posterior direction and a second curvature that is convex in the transverse direction.

4. Intervertebral implant according to claim 3, **characterised in that** the tubular peripheral wall (13) comprises lateral holes (18, 19).

5. Intervertebral implant according to one of claims 3 and 4, **characterised in that** the intermediate cylindrical wall (8) comprises peripheral holes (20-23) placing the central vertical hole (7) in communication with the peripheral through-cavities (9-12).

6. Intervertebral implant according to any one of claims 3 to 5, **characterised in that** the tubular peripheral wall (13) has, on its anterior face (6), a threaded hole (24) for holding it.

7. Intervertebral implant according to any one of claims 1 to 6, **characterised in that** the solid biocompatible material is titanium.

8. Intervertebral implant according to any one of claims 1 to 6, **characterised in that** the solid biocompatible material is a polymer of the PEEK type.

## Patentansprüche

1. Intervertebrales Implantat zur Stabilisierung von Rückenwirbeln, mit einem Körper aus festem biokompatiblem Material, der eine generell parallel epipedförmige Form hat, die durch zwei große obere (1) und untere (2) konvexe Seiten, die leicht nach vorne divergieren, durch zwei gegenüberliegende seitliche Flächen (3, 4) und durch zwei obere (5) und untere (6) Flächen begrenzt ist, mit mindestens einem inneren Hohlraum, der eine Verbindung zwischen in den großen oberen (1) und unteren (2) Flächen vorgesehenen Öffnungen herstellt,
**dadurch gekennzeichnet,**
**daß** der Körper eine hohle Struktur ist mit mehreren inneren Hohlräumen, die durch Wände begrenzt sind, mit einem mittleren vertikalen Loch (7), das von einer zylindrischen Zwischenwand (8) umgeben ist, die ihrerseits an einer rohrförmigen Umfangswand (13) mit einer vertikalen Achse durch radiale Sprossen (14-17) befestigt ist, die interne periphere durchgehende Hohlräume (9-12) begrenzen, die zu den großen oberen (1) und unteren (2) Flächen offen sind.

2. Intervertebrales Implantat nach Anspruch 1,
**dadurch gekennzeichnet,**
**daß** die großen oberen (1) und unteren (2) Flächen transversale Rippen (25) und Nuten (26) aufweisen.

3. Intervertebrales Implantat nach einem der Ansprüche 1 oder 2, **dadurch gekennzeichnet,**
**daß** die großen oberen (1) und unteren (2) Flächen eine erste konvexe Krümmung in der Richtung von vorne nach hinten und eine zweite konvexe Krümmung in der transversalen Richtung aufweisen.

4. Intervertebrales Implantat nach Anspruch 3, **dadurch gekennzeichnet,**
**daß** die rohrförmige periphere Wand (13) seitliche Löcher (18, 19) aufweist.

5. Intervertebrales Implantat nach einem der Ansprüche 3 oder 4, **dadurch gekennzeichnet,**
**daß** die zylindrische Zwischenwand (8) periphere Löcher (20-23) aufweist, die das mittlere vertikale Loch (7) mit den peripheren durchgehenden Hohlräumen (9-12) verbinden.

6. Intervertebrales Implantat nach irgendeinem der Ansprüche 3 bis 5, **dadurch gekennzeichnet,**
**daß** die rohrförmige periphere Wand (13) auf ihrer vorderen Seite (6) ein Gewindeloch (24) zum Greifen aufweist.

7. Intervertebrales Implantat nach irgendeinem der Ansprüche 1 bis 6, **dadurch gekennzeichnet,**
**daß** das feste biokompatible Material Titan ist.

8. Intervertebrales Implantat nach irgendeinem der Ansprüche 1 bis 6, **dadurch gekennzeichnet,**
**daß** das feste biokompatible Material ein Polymer des Typs PEEK ist.
